# EUROPEAN PATENT APPLICATION

(11) **EP 3 614 391 A1**
(43) Date of publication of application: **26.02.2020**
(21) Application number: 18190487.1
(22) Date of filing: 23.08.2018
(51) Int. Cl.: G16H 30/40, G16H 40/67, G16H 40/63, G06Q 50/00

(54) **MONITORING OPERATORS BASED ON DRONES**

(71) Applicant: Rain Carbon GmbH, 44579 Castrop-Rauxel (DE)
(72) Inventor: D'HONDT, Bram Sebastiaan Kristiaan, 3960 Bree (BE)
(74) Representative: Cohausz & Florack

(57) **Abstract**

A method is disclosed comprising: obtaining gathered or gathering one or more pieces of image information, wherein at least one piece of the one or more pieces of image information at least partially represents at least a part of at least one operator residing at an area of a venue, wherein the obtaining or the gathering is performed by at least one drone that continuously follows the at least one operator at least during a task the at least one operator performs; determining vital information indicative of a vitality of the at least one operator, wherein the vital information represents whether or not the at least one operator is in a safe condition or not, wherein the vital information is determined based at least partially on the obtained or gathered one or more pieces of image information; and outputting the determined vital information. Further disclosed are a corresponding apparatus, a corresponding system and a corresponding computer program.

## Description

### FIELD OF THE DISCLOSURE

The invention relates to the field of monitoring, and in particular relates to monitoring with respect to determining whether or not an operator at a location performing tasks, e.g. at a (un)loading facility, is in an unsafe condition.

### BACKGROUND

Nowadays different approaches exist to determine whether or not operators at e.g. (un)loading facilities are in an unsafe condition. For instance, one or more cameras are installed monitoring all locations of such (un)loading facilities so it can be provided a coverage of the locations 24h on 7 days per week. It is obvious that this monitoring is an elaborate process since not all of the time operators are resided at such (un)loading facilities. Further, such a monitoring leads to a huge number of screens that need to be observed by further persons, e.g. to check if all is fine. Also, many screens are live where nothing is happening.

Further, biological threshold values (BGW, also referred to as biological workplace tolerance values (BAT)), e.g. defined by the legislator) solutions are known that can be used to monitor (un)loading facilities. The environment of such (un)loading facilities are monitored e.g. by air sensors, wherein information obtained by such sensors is analyzed whether or not biological values, e.g. values of hazardous chemical agents, exceed certain limits in the environment. Such a monitoring is expensive due to a high initial invest and further for operating costs (e.g. repair and maintenance).

### SUMMARY OF SOME EXAMPLE EMBODIMENTS OF THE INVENTION

It is thus, inter alia, an object of the present invention to provide a solution in which a monitoring of operators, e.g. at (un)loading facilities can be performed at least partially automatically while ensuring that the monitoring covers all necessary information to determine whether or not the operator(s) is/are in an unsafe condition.

According to a first exemplary aspect of the invention, a method is disclosed, the method comprising:
- obtaining gathered or gathering one or more pieces of image information, wherein at least one piece of the one or more pieces of image information at least partially represents at least a part of at least one operator residing at an area of a venue, wherein the obtaining or the gathering is performed by at least one drone that continuously follows the at least one operator at least during a task the at least one operator performs;
- determining vital information indicative of a vitality of the at least one operator, wherein the vital information represents whether or not the at least one operator is in a safe condition or not, wherein the vital information is determined based at least partially on the obtained or gathered one or more pieces of image information; and
- outputting the determined vital information.

This method may for instance be performed and/or controlled by an apparatus, for instance a drone. Alternatively, this method may for instance be performed and/or controlled by an apparatus, for instance a server. Alternatively, this method may for instance be performed and/or controlled by a plurality of apparatuses, for instance a drone and a server.

In some exemplary embodiments of the present invention, some method steps of the method according to the first exemplary aspect of the present invention may thus be performed and/or controlled by the server, the server cloud, or the terminal (e.g. determining the vital information). Other method steps (e.g. gathering of the one or more pieces of image information) of the method according to the first exemplary aspect of the present invention may for instance be performed and/or controlled by the at least one apparatus (e.g. a drone). In some example embodiments, the at least one apparatus (e.g. a drone) may for instance be communicating with the server or the server cloud and/or the terminal.

According to a further exemplary aspect of the invention, a computer program is disclosed, the computer program when executed by a processor causing an apparatus, for instance a drone, to perform and/or control the actions of the method according to the first exemplary aspect.

The computer program may be stored on computer-readable storage medium, in particular a tangible and/or non-transitory medium. The computer readable storage medium could for example be a disk or a memory or the like. The computer program could be stored in the computer readable storage medium in the form of instructions encoding the computer-readable storage medium. The computer readable storage medium may be intended for taking part in the operation of a device, like an internal or external memory, for instance a Read-Only Memory (ROM) or hard disk of a computer, or be intended for distribution of the program, like an optical disc.

According to a further exemplary aspect of the invention, an apparatus is disclosed, configured to perform and/or control or comprising respective means for performing and/or controlling the method according to the first exemplary aspect.

The means of the apparatus can be implemented in hardware and/or software. They may comprise for instance at least one processor for executing computer program code for performing the required functions, at least one memory storing the program code, or both. Alternatively, they could comprise for instance circuitry that is designed to implement the required functions, for instance implemented in a chipset or a chip, like an integrated circuit. In general, the means may comprise for instance one or more processing means or processors.

According to a further exemplary aspect of the invention, an apparatus is disclosed, comprising at least one processor and at least one memory including computer program code, the at least one memory and the computer program code configured to, with the at least one processor, cause an apparatus, for instance the apparatus, at least to perform and/or to control the method according to the first exemplary aspect.

The above-disclosed apparatus according to any aspect of the invention may be a module or a component for a device, for example a chip. Alternatively, the disclosed apparatus according to any aspect of the invention may be a device, for instance be comprised by a drone. The disclosed apparatus according to any aspect of the invention may comprise only the disclosed components, for instance means, processor, memory, or may further comprise one or more additional components.

According to a further exemplary aspect of the invention, a system is disclosed, comprising at least one apparatus according to any aspect of the invention as disclosed above, and a server, a server-cloud (e.g. more than one apparatuses, for instance at least two servers communicating with each other over a communication network, e.g. the Internet), and/or a terminal, wherein the at least one apparatus and the server, the server cloud, and/or the terminal are configured to perform and/or control the method according to the first exemplary aspect of the present invention together.

For instance, in an example embodiment, the server may be configured to receive the determined vital information, e.g. from the at least one apparatus according to any aspect of the invention as disclosed above, and further, the server may be configured to receive an input indicative of an acknowledgement of the received vital information, e.g. from the terminal that is different from the at least one apparatus.

In the following, exemplary features and exemplary embodiments of all aspects of the present invention will be described in further detail.

The venue may for instance be a chemical fabric, explosive endangered area, security area, area with danger of enhanced radiation or the like to name but a few non-limiting examples.

As used herein, the term "drone" may refer to an (e.g. autonomous and/or remote controlled) unmanned aerial vehicle (UAV). Further, the term "drone" and/or the plural form of this term are used throughout herein to refer to any unmanned aerial system, in particular remotely controllable and/or operating autonomously. Such a drone may for instance be operated by a provider of the area, or the venue, or a third party.

According to example embodiments of the present invention, the at least one drone moves (e.g. flies) independent of control signals of a human operator. Such a drone within the meaning of the present invention is also referred to as autonomous drone respectively device. For being autonomous, the drone may for instance comprise a controller, e.g. which may control (e.g. move respectively fly) the drone according to one or more control and/or direction information (more details with respect to those information are described below). The moving respectively flying of the at least one drone may for instance be based at least partially, and besides the features and aspects described in conjunction with the present invention, on one or more sensor information, e.g. gathered (e.g. measured) by one or more sensors that are comprised by or being connectable to the at least one drone. Each of the one or more sensor information may for instance be indicative of an information with respect to one or more certain parameters of the surrounding of the at least one drone that enable the at least one drone to (e.g. autonomously) move (e.g. fly) or navigate according to the requirements set out by the surrounding of the at least one drone.

The drone may for instance comprise or be connectable to a memory, a communication interface, at least one sensor (e.g. environmental sensor; additionally an optical sensor, like a camera, to name but one non-limiting example), and a processor configured with processor-executable code to perform operations of the method according to the first exemplary aspect of the present invention. An exemplary embodiment of the apparatus performing the method according to the first exemplary aspect of the present invention may for instance comprise or include respective means for performing and/or controlling at least partially the steps of the method according to the first exemplary aspect of the present invention.

The at least one operator may for instance be a human operator, e.g. working at the area of the venue. The at least one operator may for instance perform a task, e.g. associated with or required at the area and/or the venue, e.g. a loading and/or unloading of chemical agents. Such an area of the venue may for instance be a loading or unloading facility, wherein hazardous goods may be handled by the at least one operator. Such hazardous goods may for instance be chemical agents, e.g. chemical agents that are volatile. Such a volatile chemical agent may for instance be of physical state i) solid, ii) liquid, and/or gaseous.

Such a chemical agent may for instance have a boiling point range in degree Celsius (°C) of <0 to 50-100; 50-100 to 240-260; and/or 240-260 to 380-400. Such a chemical agent may for instance be very volatile (gaseous) organic compounds (VVOC), or semi volatile organic compounds (SVOC). Such a chemical agent may for instance be nitrogen oxide (NOx), or sulfur oxide (SOx).

In some example embodiments of the present invention, additionally, more than one operators may for instance be present at the area of the venue, e.g. work together.

In this case of more than one operators, the one or more pieces of image information may for instance represent at least partially the more than one operators, e.g. simultaneously (e.g. by one piece of image information representing more than one operators).

A respective piece of image information may for instance be a digital image taken, e.g. by a sensor. More details with respect to such a sensor is described in the following. A respective piece of image information may thus be represented by a still image. In case more than one piece (e.g. a plurality of pieces) of image information are obtained or gathered, those more than one pieces of image information may thus be represented by a video sequence.

The gathering of the one or more pieces of image information may for instance be performed by one or more sensors, e.g. that are comprised by the apparatus that performs and/or controls the method according to the first exemplary aspect of the present invention. For instance, each respective piece of image information of the one or more pieces of image information may for instance be gathered by at least one sensor, e.g. an optical sensor, or image sensor, like e.g. a camera, or infrared-camera, or spectral camera, to name but a few non-limiting examples. Further, an example of such a sensor may for instance be a so-called EX Camera (EX: explosion proof) that may for instance be suitable for using e.g. in hazardous areas, e.g. the area of the venue.

The one or more pieces of image information may for instance be obtained (e.g. received) in case the method according to the first exemplary aspect is performed by a server. Then, prior to the obtaining of the one or more pieces of image information, those one or more pieces of image information are gathered, e.g. by a drone, and then be transmitted to the server.

Further, some pieces of image information of the one or more pieces of image information may for instance not represent at least a part (or the whole operator) of the at least one operator. In case that more than one pieces of image information are obtained or gathered, those more than one pieces of image information may for instance show a time sequence of the at least one operator (e.g. doing work at the area). This case is also covered by the term "continuously" as used within the meaning of the present invention.

The obtaining or the gathering may for instance be performed by at least one drone that continuously follows the at least one operator. The at least one drone is then configured to follow the operator in the way that in case the at least one operator may move, e.g. to perform work at one or more different locations that are e.g. inside the area, the at least one drone may follow him, e.g. by flying to the different location as well. Following within the meaning of the invention may refer to the at least one drone being able to gather one or more pieces of image information that represent at least a part of the at least one operator, e.g. by an image or video sequence in which the at least one operator is present. Further, the at least one drone may be configured to follow the at least one operator, e.g. by obtaining (e.g. receiving) one or more position information indicative of a current position and/or location of the at least one operator. Such a position information may for instance be transmitted, e.g. steadily or in pre-determined time intervals, by a device that the at least one operator is wearing or carries with him, e.g. while performing work at or within the area of the venue. For instance, such a device may be communicating with the at least one drone, e.g. via a wireless communication network (e.g. cellular network or Wireless Local Area Network (WLAN), to name but a few non-limiting examples).

The vital information is indicative of a vitality of the at least one operator, wherein the vital information represents whether or not the at least one operator is in a safe condition or not. The at least one operator may for instance be in a safe condition in case there is no harming happening to the at least one operator e.g. that is caused by one or more goods that the at least one operator may handle while being located (e.g. performing a task) at the area of the venue. Based on the obtained or gathered one or more pieces of image information, the vital information is determined.

In case the at least one operator is in an unsafe condition, one or more actions may for instance be needed to be performed, e.g. one or more further human operators need to be advised to check whether or not the at least one operator is safe, or as an additional or alternative action, one or more signals (e.g. acoustic, optical) may be sent, e.g. to a terminal that another operator carries with him or to a server. Additionally or alternatively, such one or more signals may for instance be sent to the at least one operator as well so that the at least one operator is requested to acknowledge that he is in a safe condition. The at least one operator may for instance be given a certain amount of time to perform (e.g. transmit back) the acknowledge. Otherwise, it may be highly likely that the operator is in an unsafe condition. Then, e.g. an environment information to name but one non-limiting example may be gathered as an action, e.g. to determine whether or not e.g. toxic parts are contained by the environment (e.g. in the air surrounding the at least one operator). Based at least partially on such an environment information, it may for instance be determined whether or not another operator that may check for the at least one operator may be required to use (e.g. wear) corresponding protective gear or wear for personal safety, to name but one non-limiting example.

The determined vital information is then output, e.g. to a server and/or a terminal, or to another apparatus that transfers the vital information to the server and/or the terminal.

The method according to the first exemplary aspect of the present invention may for instance be performed continuously. Continuously within the meaning of the present invention may for instance be a steady or at least in pre-determined or determined according to pre-defined rules time intervals. Thus, the at least one drone may monitor the at least one operator during the entire time the at least one operator is located at or within the area of the venue, e.g. because the at least one operator may perform one or more working tasks. Then, steady or at least in pre-determined or determined according to pre-defined rules time intervals, one or more pieces of image information are gathered e.g. by the at least one drone. Alternatively, the one or more pieces of image information are obtained (e.g. received), e.g. by a server from a drone. Then, the vital information is determined. Thus, the at least one drone following the at least one operator and continuously gathering one or more pieces of image information, gathered these one or more pieces of image information that may for instance represent different parts of the area (e.g. facility), or the same part of the area but from respectively different perspectives, or from different areas of the same venue, or of another area of a different venue.

Example embodiments of the invention thus make it possible to determine whether or not at least one operator working at the area, e.g. a hazardous area and the operator performs loading or unloading of hazardous goods, is in a safe condition. Thus, in case anything might happen to the at least one operator, at least partially automatically it can be determined that something has happened, and e.g. further actions to help the operator can be initiated quickly without it being required that further human operators monitor the area, manually.

According to an exemplary embodiment of all aspects of the present invention, the vital information is determined by verifying (e.g. analyzing) one or more vital signs indicative of the vitality of the at least one operator that are derived from the obtained or gathered one or more pieces of image information.

The verifying (e.g. analyzing) may for instance be performed by e.g. an image processing, or further based on gathered environment information, or a derived or determined association between image information and environment information, to name but a few non-limiting examples. More details with respect to environment information are described in the following parts of this SUMMARY section.

According to an exemplary embodiment of all aspects of the present invention, the one or more vital signs comprise or contain one or more of the following parameters i) to v) indicative of the vitality of the at least one operator:
i) heart rate or pulse of the at least one operator;
ii) movement of the at least one operator;
iii) body temperature of the at least one operator;
iv) respiratory rate of the at least one operator; and
v) pain of the at least one operator.

The movement of the at least one operator may for instance be determined, e.g. by analyzing at least two pieces of image information e.g. that are consecutively gathered. Then, it may for instance be determined whether or not the at least one operator has moved or not. Then, a further checking may for instance be performed, e.g. by comparing the movement, or e.g. the time that the at least one operator has not moved, to corresponding reference value(s) in order to determine whether or not it is likely that the at least one operator is in an unsafe condition.

The body temperature of the at least one operator may for instance be determined in case that at least one image information of the one or more pieces of image information is gathered e.g. by an infrared sensor. Based on an image information that is gathered by such an infrared sensor, the body temperature may for instance be determined.

The pain of the at least one operator may for instance be determined in case that at least one image information may for instance represent signs of pain of the at least one operator, e.g. represent by a certain facial expression, to name but one non-limiting example. Further, in case the gathered image information is associated with an acoustic information, or an acoustic information is gathered simultaneously to the respective image information, this may for instance be indicative of pain of the at least one operator as well.

The heart rate or pulse of the at least one operator or the respiratory rate of the at least one operator may for instance be determined in case the one or more pieces of image information are indicative of an unsafe condition of the at least one operator. Then, for instance information representing the heart rate or pulse of the at least one operator or the respiratory rate of the at least one operator, e.g. gathered by corresponding one or more sensors that the at least one operator may optionally wear may for instance be requested, so that the vital information can be determined further based at least partially on the corresponding information gathered by such one or more sensors that the at least one operator may optionally wear.

According to an exemplary embodiment of all aspects of the present invention, the method further comprises:
- gathering one or more pieces of operator information indicative of one or more vital signs of the operator (e.g. heartbeat, blood pressure, breathing activity), wherein the vital information is further determined based at least partially on the one or more pieces of operator information.

The heart rate or pulse of the at least one operator or the respiratory rate of the at least one operator may for instance be represented by such one or more pieces of operator information.

According to an exemplary embodiment of all aspects of the present invention, the method further comprises:
- determining or receiving at least one control information indicative of causing an apparatus that gathered the one or more pieces of image information to move corresponding to a direction that is represented or comprised by the at least one control information, wherein the at least one control information enables the apparatus to gather at least one piece of the one or more pieces of image information.

The direction may for instance be represented by a 2-dimensional or 3-dimensional direction information (e.g. in the form of corresponding coordinates, e.g. X, Y, and optionally Z-coordinates, or latitude, longitude, and optionally altitude coordinates, to name but a few non-limiting examples). For instance, based on such a direction information (e.g. that is received by the at least one drone), the at least one drone may be able or be configured to move (e.g. fly) to the coordinates that are represented by the direction information. Such a direction information may for instance be determined by a device that the at least one operator may wear or carry with him. For instance, the direction information may be determined by performing a positioning technique, e.g. determining the position based on Global Positioning System (GPS), or other techniques known from the prior art, to name but a few non-limiting examples.

According to an exemplary embodiment of all aspects of the present invention, in case more than one piece of image information are obtained or gathered, the method further comprises:
- determining one or more parts of a first image information and one or more parts of a second image information of the more than one pieces of image information that respectively represent at least a part of the at least one operator; and
- determining based at least partially on the one or more parts of the first image information and on the one or more parts of the second image information a difference between the first image information and the second image information,
   wherein the vital information is determined based at least partially on this determined difference.

The difference between the first image information and the second image information may for instance be determined, e.g. by at least selectively comparing one or more parts or the whole image represented by the first and second piece of the one or more pieces of image information to each other.

In case a difference is determined, this is indicative of that the operator has moved. So the likelihood that the operator is in a safe condition is relatively high compared to the condition in which no movement of the operator has been determined (e.g. detected).

According to an exemplary embodiment of all aspects of the present invention, the method further comprises:
- gathering one or more pieces of environment information (e.g. by a VOC detection measuring sensor, or O2 sensor) indicative of a volatile organic compound that is at least partially comprised by a probe of an environment that is represented by the gathered one or more pieces of environment information, wherein the vital information is further determined based at least partially on the one or more pieces of environment information.

Further, environment information may be considered when verifying vital signs of the at least one operator.

The at least one sensor that is configured at least to sense VOC, NOx, and/or SOx may also be referred to as at least one air pollution sensor. Such an air pollution sensor may for instance be configured to detect and/or monitor the presence of air pollution by the sense-able substance in the surrounding area (of the at least one apparatus). The at least one sensor may for instance be configured to sense (e.g. detect) gases and/or vapors, which may be oxidized (e.g. burnt). Further, such a sensor may for instance be configured to sense ozone, particulate matter, carbon monoxide, sulfur dioxide, and nitrous oxide (e.g. odors, tobacco smoke, exhalations emitted by materials, to name but a few non-limiting examples).

According to an exemplary embodiment of all aspects of the present invention, one or more pieces of image information are respectively associated with the one or more pieces of environment information, wherein the vital information is determined considering these respective one or more associations between the one or more pieces of image information and the one or more pieces of environment information

One or more pieces of image information may for instance be respectively associated with the one or more pieces of environment information by being incorporated in the image information so that the corresponding environmental information is shown in the image represented by the corresponding image information. For instance, the location of a hazardous chemical agent according to the environmental information can be derived.

In this way, each piece of the one or more pieces of environment information may for instance be characterized by a VOC (volatile organic compound) measurement, an environment measurement, an atmosphere measurement, an O2 measurement, or a combination thereof.

According to an exemplary embodiment of all aspects of the present invention, the one or more pieces of image information form a video sequence that is analyzed with respect to recognizing whether or not one or more abnormal situations are happening and/or are happening to the at least one operator by comparing the video sequence to one or more pieces of reference information, wherein each piece of the one or more pieces of reference information is indicative of such an abnormal situation that can happen and/or that can happen to the at least one operator.

The vital information is determined further by analyzing the video material (e.g. video sequence). It may for instance be able to recognize if abnormal situations are happening, e.g. by comparing the video material (e.g. video sequence, also referred to as video information) with corresponding reference information. Such reference information may for instance be obtained prior to performing the method according to the first exemplary aspect of the present invention. Such reference information may for instance be stored in a memory, e.g. a database that is comprised or being connectable to the apparatus (e.g. the at least one drone) that performs and/or controls the method according to the first exemplary aspect of the present invention.

According to an exemplary embodiment of all aspects of the present invention, the method is performed and/or controlled for more than one locations simultaneously.

In case that the method according to the first exemplary aspect of the present invention is performed and/or controlled for more than one locations (e.g. area of the venue), this method may for instance be performed in several iterations, simultaneously, e.g. one iteration for each location of the more than one locations.

A respective location may be located in the same area (of the venue), or be the same venue, but might as well be an area of a venue that is different from the venue.

In this way, a continuous monitoring of more than one (e.g. a plurality of) operators performing dangerous tasks simultaneously at more than one (e.g. a plurality of) locations is enabled.

According to an exemplary embodiment of all aspects of the present invention, the gathering of the one or more pieces of image information is performed by at least one drone.

Alternatively the actions of analyzing are performed by an apparatus that is different from the apparatus that gathered the one or more pieces of image information, e.g. a server or a terminal.

In case the method is performed for more than one location simultaneously, a plurality of drones may for instance be used, e.g. one respective drone of the plurality of drones is associated with one respective operator residing at a certain location of the more than one locations (e.g. a plurality of locations).

In particular, at least the step of gathering the one or more pieces of image information is performed by the at least one drone. Further, the at least one drone may for instance further perform the following steps:
- receiving one or more pieces of position information (e.g. from an apparatus that is carried by the at least one operator), wherein this apparatus is communicating with the at least one drone to cause the at least one drone to move (e.g. fly) corresponding to the position (additionally or alternatively direction or relative direction with respect to the apparatus that is carried by the operator) information.

Additionally or alternatively, the apparatus that is carried by the operator is communicating with the at least one drone, e.g. by transmitting one or more positioning information indicative of the position of the device to the drone, and wherein the drone moves based at least partially on received one or more positioning information

According to an exemplary embodiment of all aspects of the present invention, the vital information is output to at least one server, and/or to at least one terminal.

The at least one terminal within the meaning of the present invention may for instance be a terminal that is carried by other person than the at least one operator performing the work at the venue (e.g. loading/unloading).

The features and example embodiments of the invention described above may equally pertain to the different aspects according to the present invention.

It is to be understood that the presentation of the invention in this section is merely by way of examples and non-limiting.

Other features of the invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not drawn to scale and that they are merely intended to conceptually illustrate the structures and procedures described herein.

### BRIEF DESCRIPTION OF THE FIGURES

In the figures show:
- Fig. 1: a schematic block diagram of a system according to an exemplary aspect of the present invention;
- Fig. 2: a flowchart showing an example embodiment of a method according to the first exemplary aspect of the present invention, for instance performed by drone 130 of Fig. 1; and
- Fig. 3: a schematic block diagram of an apparatus according to an exemplary aspect of the present invention.

### DETAILED DESCRIPTION OF EXAMPLE EMBODIMENTS OF THE INVENTION

The following description serves to deepen the understanding of the present invention and shall be understood to complement and be read together with the description of the invention as provided in the above SUMMARY section of this specification.

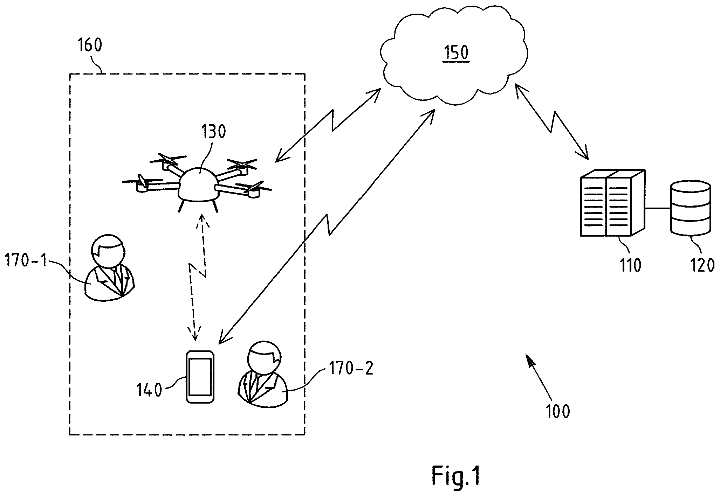

Fig. 1 is a schematic high-level block diagram of a system 100 according to an exemplary aspect of the present invention. System 100 comprises a drone 130, an optional server 110, which may alternatively be embodied as a server cloud (e.g. a plurality of servers connected e.g. via the internet and providing services at least partially jointly), an optional database 120, and an optional terminal 140. Further, system 100 comprises an optional communication network 150, which may provide communication between the entities comprised by the system 100.

Communication between at least two of the different entities of the system 100 may for instance take place at least partially in a wireless fashion, e.g. based on cellular radio communication or on Wireless Local Area Network (WLAN) based communication, to name but a few non-limiting examples. In this way, mobility of drone 130 is guaranteed.

In Fig. 1, drone 130, terminal 140 of operator 170-2 and operator 170-1 that is followed by drone 130 are located in area 160. Area 160 may for instance be part of a venue. Further, area 160 may for instance be a hazardous area, in that e.g. loading and unloading of hazardous goods may for instance be performed by operator 170-1. Drone 130 follows operator 170-1 and e.g. determines a vital information continuously, wherein the vital information is indicative of whether or not operator 170-1 is in a safe condition. In case that such a vital information represents that operator 170-1 is in an unsafe condition, the vital information may for instance be output, e.g. to server 110, and/or to terminal 140 of operator 170-2. In case vital information is output to terminal 140 of operator 170-2, operator 170-2 may for instance perform one or more actions to help operator 170-1.

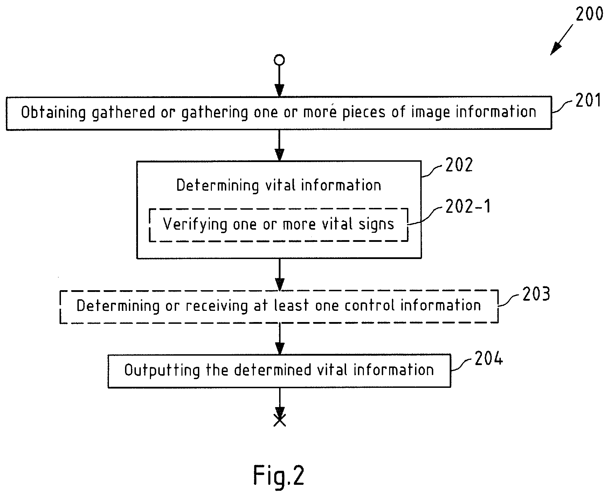

Fig. 2 is a flowchart 200 showing an example embodiment of a method according to the first exemplary aspect of the present invention. This flowchart may for instance be performed by drone 130 of Fig. 1. Alternatively, the flowchart 200 may for instance be performed by drone 130 and server 110 of Fig. 1 together.

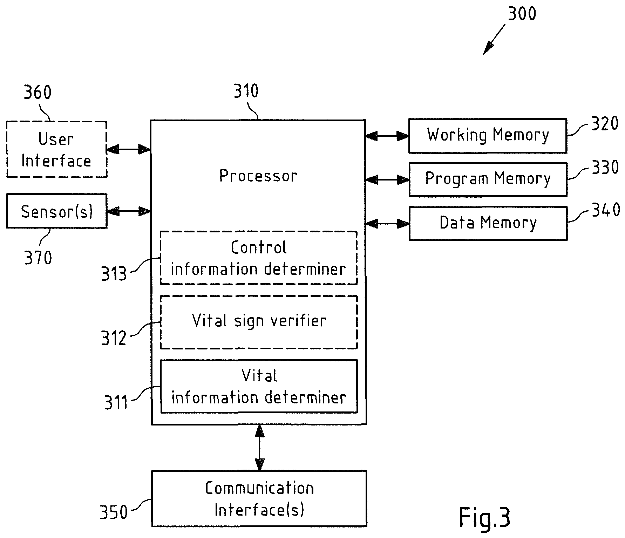

In a first step 201, one or more pieces of image information are obtained or gathered. In case this step 201 is performed and/or controlled by the drone 130, the one or more pieces of image information are obtained, e.g. by one or more sensors (e.g. sensors 370 of Fig. 3) that are comprised by the drone 130 or that are connectable to the drone 130. In case this step 201 is performed and/or controlled by the server 110, the one or more pieces of image information are obtained (e.g. received) by the server 110, e.g. from at least one drone (e.g. drone 130 of Fig. 1) that gathered the one or more pieces of image information prior to the obtaining by server 110. For instance, after gathering the one or more pieces of image information by the drone 130, the drone 130 may for instance relay the obtained one or more pieces of image information to the server 110, so that the server 110 can obtain (e.g. receive) those one or more pieces of image information.

In a second step 202, the vital information is determined. In case this step 202 is performed and/or controlled by the drone 130, the vital information is determined by the drone 130, in particular by a processor (e.g. processor 310 comprising functional or structural unit 311 of Fig. 3) comprised by the drone 130. In case this step 202 is performed and/or controlled by the server 110, the vital information is determined by the server 110, in particular by a processor (e.g. processor of the server 110 represented by an apparatus, e.g. apparatus 300 of Fig. 3, comprising a processor 310 with functional or structural unit 311 of Fig. 3).

The determining of the vital information in step 202 may for instance further comprise optional step 202-1. In this optional step 202-1, one or more vital signs of at least one operator that is monitored by at least one drone (e.g. drone 130 of Fig. 1) that gathered the one or more pieces of image information in step 201 is verified (e.g. by analyzing the obtained or gathered one or more pieces of image information of step 201). Based e.g. on the verifying, the vital information is determined correspondingly, e.g. the determined vital information may for instance represent that the at at least one operator is an a safe or in an unsafe condition.

In an optional third step 203, at least one control information is determined or received. The at least one control information is received in case this step 203 is performed and/or controlled by the drone 130. In this case, in step 201 this drone 130 also gathered the one or more pieces of image information. The at least one control information is determined in case this step 203 is performed and/or controlled by the server 110. In this latter case, the determined at least one control information is transmitted to the drone 130 that gathered the one or more pieces of image information prior to the obtaining of those one or more pieces of image information in step 201. For instance, after determining the at least one control information, the at least one control information is transmitted (e.g. sent) from the server 110 to the drone 130. Based at least partially on the at least one control information, the drone 130 may for instance move (e.g. fly) according to the at least one control information. Further, the at least one control information determined by the server 110 in the aforementioned exemplary case, may for instance be determined based at least partially on a position information that is obtained (e.g. received) by the server 110 from another entity (not shown in Fig. 1) that the at least one operator may wear or carry with him, so that based at least partially on the position information provided from this other entity to the server 110, the server 110 can determine the at least one control information. Then, the determined at least one control information is transmitted from the server 110 to the drone 130.

In a fourth step 204, the determined vital information of step 203 is then output. The determined vital information may for instance be output to the server 110 in case the step 204 is performed by drone 130 of Fig. 1, solely. Alternatively, the determined vital information may for instance be output to the terminal 130 of Fig. 1. Alternatively, the determined vital information may for instance be output to another entity that is different from the server 110 for the first mentioned case, or that is different from the terminal 140 for the latter mentioned case, wherein this entity then forwards the vital information to the server 110, or to the terminal 140.

The exemplary flowchart 200 of Fig. 2 may for instance comprise one or more further features described above, for instance a determining based at least partially on one or more parts of a first image information and one or more parts of a second image information a difference between the first image information and the second image information of the one or more pieces of image information (see step 201). Further, exemplary flowchart 200 of Fig. 2 may for instance comprise one or more further features described above, for instance a gathering of one or more pieces of operator information, and/or a gathering one or more pieces of environment information.

Furthermore, the steps 202 and 203 may be performed either in serial (as shown in Fig. 2), or in parallel. Further, the steps 201 to 203, or the step 201 to 204 may be performed continuously, e.g. steadily or in time intervals (e.g. pre-defined time intervals or determined according to pre-defined rules time intervals; for instance every 1 to 60 seconds, preferably every 2 to 10 seconds, to name but a few non-limiting examples).

Fig. 3 is a schematic block diagram of an apparatus 300 according to an exemplary aspect of the present invention, which may for instance represent the drone 130 of Fig. 1, and/or the server 110 of Fig. 1. In case apparatus 300 represents server 110 of Fig. 1, block 370 may not be comprised by such an apparatus. In case apparatus 300 represents drone 130 of Fig. 1, block 313 may not be comprised by such an apparatus.

Apparatus 300 comprises a processor 310, working memory 320, program memory 330, data memory 340, communication interface(s) 350, an optional user interface 360 and an optional sensor(s) 370.

Apparatus 300 may for instance be configured to perform and/or control or comprise respective means (at least one of 310 to 370) for performing and/or controlling the method according to the first exemplary aspect of the present invention. Apparatus 300 may as well constitute an apparatus comprising at least one processor (310) and at least one memory (320) including computer program code, the at least one memory and the computer program code configured to, with the at least one processor, cause an apparatus, e.g. apparatus 300 at least to perform and/or control the method according to the first exemplary aspect of the present invention.

Processor 310 may for instance comprise vital information determiner 311 as a functional and/or structural unit. Vital information determiner 311 may for instance be configured to determine a vital information (see step 202 of Fig. 2). Processor 310 may for instance comprise an optional vital sign verifer 312 as a functional and/or structural unit. Vital sign verifier 312 may for instance verify (e.g. analyze) one or more more pieces of image information (see step 201 of Fig. 2) on the presence of one or more indications with respect to one or more vital signs of at least one operator (see step 202-1 of Fig. 1). Processor 310 may for instance comprise an optional control information determiner 313 as a functional and/or structural unit. Control information determiner 313 may for instance be configured to determine at least one control information for a drone. In case apparatus 300 is a drone (e.g. drone 130 of Fig. 1), this apparatus 300 may as well receive at least one control information via the communication interface(s) 350, and the move (e.g. fly) in a certain direction, e.g. change altitude, roll and/or tilt the apparatus 300, and/or enable the apparatus to autonomously move according to the at least one control information (see step 203 of Fig. 2).

Processor 310 may for instance further control the memories 320 to 340, the communication interface(s) 350, the optional user interface 360 and the optional sensor(s) 370.

Processor 310 may for instance execute computer program code stored in program memory 330, which may for instance represent a computer readable storage medium comprising program code that, when executed by processor 310, causes the processor 310 to perform the method according to the first exemplary aspect of the present invention.

Processor 310 (and also any other processor mentioned in this specification) may be a processor of any suitable type. Processor 310 may comprise but is not limited to one or more microprocessor(s), one or more processor(s) with accompanying one or more digital signal processor(s), one or more processor(s) without accompanying digital signal processor(s), one or more special-purpose computer chips, one or more field-programmable gate array(s) (FPGA(s)), one or more controller(s), one or more application-specific integrated circuit(s) (ASIC(s)), or one or more computer(s). The relevant structure/hardware has been programmed in such a way to carry out the described function. Processor 310 may for instance be an application processor that runs an operating system.

Program memory 330 may also be included into processor 310. This memory may for instance be fixedly connected to processor 310, or be at least partially removable from processor 310, for instance in the form of a memory card or stick. Program memory 330 may for instance be non-volatile memory. It may for instance be a FLASH memory (or a part thereof), any of a ROM, PROM, EPROM and EEPROM memory (or a part thereof) or a hard disc (or a part thereof), to name but a few examples. Program memory 330 may also comprise an operating system for processor 310. Program memory 330 may also comprise a firmware for apparatus 300.

Apparatus 300 comprises a working memory 320, for instance in the form of a volatile memory. It may for instance be a Random Access Memory (RAM) or Dynamic RAM (DRAM), to give but a few non-limiting examples. It may for instance be used by processor 310 when executing an operating system and/or computer program.

Data memory 340 may for instance be a non-volatile memory. It may for instance be a FLASH memory (or a part thereof), any of a ROM, PROM, EPROM and EEPROM memory (or a part thereof) or a hard disc (or a part thereof), to name but a few examples. Data memory 340 may for instance store one or more pieces of image information, one or more pieces of vital information, one or more pieces of control information, one or more pieces of position information, one or more pieces of environment information, one or more pieces of operator information, to name but a few non-limiting examples. Data memory 340 may for instance store further pieces of information as described above, and/or one or more pieces of interim result information that may occur e.g. when performing and/or controlling example embodiments of the method according to the first exemplary aspect of the present invention.

Communication interface(s) 350 enable apparatus 300 to communicate with other entities, e.g. with server 110 of Fig. 1 in case apparatus 300 is represented by drone 130 of Fig. 1; or with drone 130 of Fig. 1 in case apparatus 300 is represented by server 110 of Fig. 1. The communication interface(s) 350 may for instance comprise a wireless interface, e.g. a cellular radio communication interface and/or a WLAN, Bluetooth (BT) and/or Bluetooth Low Energy (BLE) interface, for instance to communicate with entities, e.g. via communication network 150 of Fig. 1 (e.g. the Internet).

User interface 360 is optional and may comprise a display for displaying information to a user and/or an input device (e.g. a keyboard, keypad, touchpad, mouse, etc.) for receiving information from a user.

Sensor(s) 370 may for instance comprise an environmental sensor (e.g. air pollution sensor) and/or an optical sensor (e.g. a camera), to name but one non-limiting example. In particular, sensor(s) may for instance be configured at least to sense VOC, NOx, and/or SOx.

Some or all of the components of the apparatus 300 may for instance be connected via a bus. Some or all of the components of the apparatus 300 may for instance be combined into one or more modules.

The following example embodiments shall also be considered to be disclosed:
The present invention comprises the use of a drone as continuous monitoring of operators performing dangerous tasks.

Such a drone may for instance "follow" the operator(s) because he/she is wearing a device which is communicating with the drone. The drone makes sure that the operator(s) is seen (e.g. the operator is seen 100 % of the time). Then it is determined, e.g. based at least partially on a software analyzing e.g. video material and that is enabled to recognize if abnormal situations are happening. For instance, by checking of the operator still has a heartbeat, is still moving, by using a VOC detection measuring the atmosphere, O2 measurement, or the like, to name but a few non-limiting examples.

After an abnormal situation is detected, a signal is sent, e.g. to the control room.

In this way, the capex and repair and maintenance budget required can be reduced. As most of the time one single operator is only working on one single loading spot. Therefore all the other loading spots with 24/7 coverage where nothing is happening are obsolete. As the drone is following the operator, it can be ensured it is always at present or known how the operator is doing (e.g. whether or not the operator is in a safe condition).

Further, the operator can be monitored by such a drone 100 % of the operator time by the drone following the operator.

In the present specification, any presented connection in the described embodiments is to be understood in a way that the involved components are operationally coupled. Thus, the connections can be direct or indirect with any number or combination of intervening elements, and there may be merely a functional relationship between the components.

Moreover, any of the methods, processes and actions described or illustrated herein may be implemented using executable instructions in a general-purpose or special-purpose processor and stored on a computer-readable storage medium (e.g., disk, memory, or the like) to be executed by such a processor. References to a 'computer-readable storage medium' should be understood to encompass specialized circuits such as FPGAs, ASICs, signal processing devices, and other devices.

The expression "A and/or B" is considered to comprise any one of the following three scenarios: (i) A, (ii) B, (iii) A and B. Furthermore, the article "a" is not to be understood as "one", i.e. use of the expression "an element" does not preclude that also further elements are present. The term "comprising" is to be understood in an open sense, i.e. in a way that an object that "comprises an element A" may also comprise further elements in addition to element A.

It will be understood that all presented embodiments are only exemplary, and that any feature presented for a particular example embodiment may be used with any aspect of the invention on its own or in combination with any feature presented for the same or another particular example embodiment and/or in combination with any other feature not mentioned. In particular, the example embodiments presented in this specification shall also be understood to be disclosed in all possible combinations with each other, as far as it is technically reasonable and the example embodiments are not alternatives with respect to each other. It will further be understood that any feature presented for an example embodiment in a particular category (method/apparatus/computer program/system) may also be used in a corresponding manner in an example embodiment of any other category. It should also be understood that presence of a feature in the presented example embodiments shall not necessarily mean that this feature forms an essential feature of the invention and cannot be omitted or substituted.

The statement of a feature comprises at least one of the subsequently enumerated features is not mandatory in the way that the feature comprises all subsequently enumerated features, or at least one feature of the plurality of the subsequently enumerated features. Also, a selection of the enumerated features in any combination or a selection of only one of the enumerated features is possible. The specific combination of all subsequently enumerated features may as well be considered. Also, a plurality of only one of the enumerated features may be possible.

The sequence of all method steps presented above is not mandatory, also alternative sequences may be possible. Nevertheless, the specific sequence of method steps exemplarily shown in the figures shall be considered as one possible sequence of method steps for the respective embodiment described by the respective figure.

The invention has been described above by means of example embodiments. It should be noted that there are alternative ways and variations which are obvious to a skilled person in the art and can be implemented without deviating from the scope of the appended claims.

## Claims

1. Method, comprising:
- obtaining gathered or gathering one or more pieces of image information,
wherein at least one piece of the one or more pieces of image information at least partially represents at least a part of at least one operator residing at an area of a venue, wherein the obtaining or the gathering is performed by at least one drone that continuously follows the at least one operator at least during a task the at least one operator performs;
- determining vital information indicative of a vitality of the at least one operator,
wherein the vital information represents whether or not the at least one operator is in a safe condition or not, wherein the vital information is determined based at least partially on the obtained or gathered one or more pieces of image information; and
- outputting the determined vital information.

2. The method according to claim 1, wherein the vital information is determined by verifying one or more vital signs indicative of the vitality of the at least one operator that are derived from the obtained or gathered one or more pieces of image information.

3. The method according to claim 2, wherein the one or more vital signs comprise or contain one or more of the following parameters i) to v) indicative of the vitality of the at least one operator:
i) heart rate or pulse of the at least one operator;
ii) movement of the at least one operator;
iii) body temperature of the at least one operator;
iv) respiratory rate of the at least one operator; and
v) pain of the at least one operator.

4. The method according to any of the preceding claims, wherein the method further comprises:
- determining or receiving at least one control information indicative of causing an apparatus that gathered the one or more pieces of image information to move corresponding to a direction that is represented or comprised by the at least one control information, wherein the at least one control information enables the apparatus to gather at least one piece of the one or more pieces of image information.

5. The method according to any of the preceding claims, wherein in case more than one piece of image information are obtained or gathered, the method further comprises:
- determining one or more parts of a first image information and one or more parts of a second image information of the more than one pieces of image information that respectively represent at least a part of the at least one operator; and
- determining based at least partially on the one or more parts of the first image information and on the one or more parts of the second image information a difference between the first image information and the second image information,
wherein the vital information is determined based at least partially on this determined difference.

6. The method according to any of the preceding claims, wherein the method further comprises:
- gathering one or more pieces of environment information indicative of a volatile organic compound that is at least partially comprised by a probe of an environment that is represented by the gathered one or more pieces of environment information, wherein the vital information is further determined based at least partially on the one or more pieces of environment information.

7. The method according to claim 6, wherein one or more pieces of image information are respectively associated with the one or more pieces of environment information, wherein the vital information is determined considering these respective one or more associations between the one or more pieces of image information and the one or more pieces of environment information

8. The method according to any of the preceding claims, wherein the one or more pieces of image information form a video sequence that is analyzed with respect to recognizing whether or not one or more abnormal situations are happening and/or are happening to the at least one operator by comparing the video sequence to one or more pieces of reference information, wherein each piece of the one or more pieces of reference information is indicative of such an abnormal situation that can happen and/or that can happen to the at least one operator.

9. The method according to any of the preceding claims, wherein the method further comprises:
- gathering one or more pieces of operator information indicative of one or more vital signs of the operator, wherein the vital information is further determined based at least partially on the one or more pieces of operator information.

10. The method according to any of the preceding claims, wherein the method is performed and/or controlled for more than one location simultaneously.

11. The method according to any of the preceding claims, wherein the gathering of the one or more pieces of image information is performed by at least one drone.

12. The method according to any of the preceding claims, wherein the vital information is output to at least one server, and/or to at least one terminal.

13. A computer program, the computer program when executed by a processor causing an apparatus to perform and/or control the actions of the method of any of the preceding claims.

14. An apparatus configured to perform and/or control or comprising respective means for performing and/or controlling the method of any of the claims 1 to 12.

15. A system comprising:
- a drone according to claim 14; and
- a server, wherein the server is configured to receive the determined vital information and to receive an input indicative of an acknowledgement of the received vital information, wherein the input is received from a terminal that is different from the first apparatus.
